# EUROPEAN PATENT APPLICATION

(11) **EP 4 216 152 A1**
(43) Date of publication of application: **26.07.2023**
(21) Application number: 21868713.5
(22) Date of filing: 17.09.2021
(51) Int. Cl.: G06T 7/00, G06T 7/62, A61B 5/00

(54) **ORAL CAVITY TEST METHOD, ORAL CAVITY TEST DEVICE, AND COMPUTER-READABLE STORAGE MEDIUM**

(30) Priority: 18.09.2020 CN 202010986802
(71) Applicant: Johnson & Johnson Consumer Inc., Skillman, NJ 08558 (US)
(72) Inventor: LIU, Fengxing, Shanghai 200245 (CN); YAN, Erping, Shanghai 200245 (CN); WU, Qingbin, Shanghai 201306 (CN)
(74) Representative: Carpmaels & Ransford LLP
(86) International application number: PCT/CN2021/119037
(87) International publication number: WO 2022/057891

(57) **Abstract**

The present disclosure provides an oral cavity detection method. The method comprises: acquiring an ultraviolet light image of the oral cavity; determining a first region including a tooth region and at least one portion of a gum region based on the ultraviolet light image; acquiring a non-ultraviolet light image of the oral cavity; acquiring from the non-ultraviolet light image a first intermediate image that only includes the corresponding first region of the non-ultraviolet light image, based on the non-ultraviolet light image and the first region; determining a second region that only includes the tooth region, based on the first intermediate image; and determining the proportion of pixels containing dental plaque in a second intermediate image that only includes the corresponding second region of the ultraviolet light image. Through the combined use of ultraviolet light and non-ultraviolet light images, the oral cavity detection method proposed by one embodiment according to the present disclosure can accurately determine a tooth region, and thereby can accurately determine the proportion of pixels containing dental plaque, so as to provide a favorable basis for dental plaque assessment.

## Description

### TECHNICAL FIELD

The present disclosure relates to the field of oral cavity detection, and more specifically to an oral cavity detection method, an oral cavity detection device, and a computer-readable storage medium for executing the above oral cavity detection method.

### BACKGROUND

US2019/0167115 A1 discloses a system for processing an image of an oral cavity, including an image capture device; a mouthpiece attachment, configured to attach to the image capture equipment and mount the image capture equipment in a fixed position relative to a user's mouth; a display; and a image processing device, wherein the image processing device is configured to: receive mouth image data from the image capture equipment; identify an region of analysis within the image data corresponding to teeth and gums; extract a set of image property features from the region of analysis of the image data; pass the image property features through a condition classifier which is configured to compare the values of the extracted image property features against pre-set parameters to identify sub-regions of the region of analysis which are indicative of oral health conditions; and send result data corresponding to the identified sub-regions to the display.

### SUMMARY

As mentioned above, the prior art suffers from the following technical problems. The traditional method of analyzing dental plaque by ultraviolet light only adopts ultraviolet light images for analysis, and the oral cavity images under ultraviolet light are presented in a fluorescent color. At this moment, teeth and gums both exhibit a fluorescent color and cannot be distinguished well, leading to inaccurate detection.

The present disclosure aims to distinguish the tooth region, and then perform targeted detection on dental plaque. In view of the above technical problems, a first aspect of the present disclosure proposes an oral cavity detection method, where the oral cavity detection method comprises:
acquiring an ultraviolet light image of the oral cavity;
determining a first region comprising a tooth region and at least one portion of a gum region based on the ultraviolet light image;
acquiring a non-ultraviolet light image of the oral cavity;
acquiring from the non-ultraviolet light image a first intermediate image that only comprises the corresponding first region of the non-ultraviolet light image, based on the non-ultraviolet light image and the first region;
determining a second region that only comprises the tooth region, based on the first intermediate image; and
determining the proportion of pixels containing dental plaque in a second intermediate image that only comprises the corresponding second region of the ultraviolet light image.

Through the combined use of ultraviolet light and non-ultraviolet light images, the oral cavity detection method proposed by one embodiment according to the present disclosure can accurately determine teeth and tooth regions, and thereby can accurately determine the proportion of pixels containing dental plaque, so as to provide a favorable basis for dental plaque assessment.

In one embodiment according to the present disclosure, the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

In one embodiment according to the present disclosure, the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

In one embodiment according to the present disclosure, the determination of the first region comprising the tooth region and the at least one portion of the gum region based on the ultraviolet light image further comprises:
adjusting the contrast of the ultraviolet light image.

In one embodiment according to the present disclosure, the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

In one embodiment according to the present disclosure, the determination of the second region that only comprises the tooth region based on the first intermediate image further comprises:
adjusting the contrast of the first intermediate image.

In one embodiment according to the present disclosure, the non-ultraviolet light is white light or yellow light.

In addition, a second aspect of the present disclosure proposes an oral cavity detection device, where the oral cavity detection device comprises:
a non-ultraviolet light module, configured to emit non-ultraviolet light;
an ultraviolet light module, configured to emit ultraviolet light;
a camera device, configured to acquire a non-ultraviolet light image of the oral cavity and an ultraviolet light image of the oral cavity; and
a image processing device, configured to determine the proportion of pixels containing dental plaque based on the non-ultraviolet light image and the ultraviolet light image.

In one embodiment according to the present disclosure, the image processing device is further configured to:
acquire an ultraviolet light image of the oral cavity;
determine a first region comprising a tooth region and at least one portion of a gum region based on the ultraviolet light image;
acquire a non-ultraviolet light image of the oral cavity;
acquire from the non-ultraviolet light image a first intermediate image that only comprises the corresponding first region of the non-ultraviolet light image, based on the non-ultraviolet light image and the first region;
determine a second region that only comprises the tooth region, based on the first intermediate image; and
determine the proportion of pixels containing dental plaque in a second intermediate image that only comprises the corresponding second region of the ultraviolet light image.

In one embodiment according to the present disclosure, the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
   determining the proportion of pixels containing dental plaque based on the second intermediate image converted to the HSV color space and the dental plaque comparison threshold.

In one embodiment according to the present disclosure, the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

In one embodiment according to the present disclosure, the determination of the first region comprising the tooth region and the at least one portion of the gum region based on the ultraviolet light image further comprises:
adjusting the contrast of the ultraviolet light image.

In one embodiment according to the present disclosure, the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

In one embodiment according to the present disclosure, the determination of the second region that only comprises the tooth region based on the first intermediate image further comprises:
adjusting the contrast of the first intermediate image.

In one embodiment according to the present disclosure, the non-ultraviolet light is white light or yellow light.

Moreover, a third aspect of the present disclosure proposes a tangible computer-readable storage medium, where the storage medium comprises an instruction for executing the oral cavity detection method, and when the instruction is executed, a processor of the computer is configured at least to:
acquire an ultraviolet light image of the oral cavity;
determine a first region comprising a tooth region and at least one portion of a gum region based on the ultraviolet light image;
acquire a non-ultraviolet light image of the oral cavity;
acquire from the non-ultraviolet light image a first intermediate image that only comprises the corresponding first region of the non-ultraviolet light image, based on the non-ultraviolet light image and the first region;
determine a second region that only comprises the tooth region, based on the first intermediate image; and
determine the proportion of pixels containing dental plaque in a second intermediate image that only comprises the corresponding second region of the ultraviolet light image.

In one embodiment according to the present disclosure, the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
   determining the proportion of pixels containing dental plaque based on the second intermediate image converted to the HSV color space and the dental plaque comparison threshold.

In one embodiment according to the present disclosure, the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

In one embodiment according to the present disclosure, the determination of the first region comprising the tooth region and the at least one portion of the gum region based on the ultraviolet light image further comprises:
adjusting the contrast of the ultraviolet light image.

In one embodiment according to the present disclosure, the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

In one embodiment according to the present disclosure, the determination of the second region that only comprises the tooth region based on the first intermediate image further comprises:
adjusting the contrast of the first intermediate image.

In one embodiment according to the present disclosure, the non-ultraviolet light is white light or yellow light.

In summary, through the combined use of ultraviolet light and non-ultraviolet light images, the oral cavity detection method, the oral cavity detection device and correspondingly the computer-readable storage medium provided by the three aspects of the present disclosure can accurately determine teeth and junction regions between teeth and gums, and thereby can accurately determine the proportion of pixels containing dental plaque, so as to provide a favorable basis for dental plaque assessment. Other advantages of the present disclosure are further described below.

### BRIEF DESCRIPTION OF THE DRAWINGS

With reference to the following detailed description in combination with the accompanying drawings, the features, advantages, and other aspects of the embodiments of the present disclosure become more obvious. Several embodiments of the present disclosure are shown in an exemplary rather than restrictive manner herein. Regarding the accompanying drawings:
FIG. 1 shows a flowchart of oral cavity detection method 100 according to an embodiment of the present disclosure;
FIG. 2 shows a schematic block diagram of oral cavity detection device 200 according to an embodiment of the present disclosure; and
FIG. 3 shows a schematic block diagram of oral cavity detection device 300 according to another embodiment of the present disclosure.

### DETAILED DESCRIPTION

Various exemplary embodiments of the present disclosure are described in detail below with reference to the accompanying drawings. Although the exemplary methods and devices described below include software and/or firmware executed on hardware in other components, it should be noted that these examples are merely illustrative and should not be considered limiting. For example, it is contemplated that any or all hardware, software, and firmware components may be implemented exclusively in hardware, exclusively in software, or in any combination of hardware and software. Accordingly, although the exemplary methods and devices are described below, it should be readily understood by those skilled in the art that the examples provided are not intended to limit manners for implementing these methods and devices.

Moreover, the flowcharts and block diagrams in the accompanying drawings illustrate a system architecture, a function, and an operation that can be possibly implemented by the methods and systems according to various embodiments of the present disclosure. It should be noted that the functions noted in the blocks may also occur in an order different from that noted in the drawings. For example, two blocks represented in succession may actually be substantially executed in parallel, or they may sometimes be executed in the reverse order, depending upon the function involved. It should also be noted that each block of the flowcharts and/or block diagrams, and combinations of blocks in the flowcharts and/or block diagrams, may be implemented using dedicated hardware-based systems that perform the specified functions or operations, or may be implemented using a combination of dedicated hardware and computer instructions.

The term "median filtering" in the present disclosure basically refers to a nonlinear signal processing technology that can effectively suppress noise based on the order statistical theory. The basic principle of median filtering is that, the value of a point in a digital image or digital sequence is replaced by the median value of each point in a neighborhood of the point so that the surrounding pixel values are close to the true values, thereby eliminating isolated noise points. Where the pixel point refers to the point constituting the image in a unit area, and where each pixel point may have a different color value. The more pixels per unit area, the higher the resolution and the better the image effect.

The term "adaptive threshold segmentation" in the present disclosure basically refers to an image processing technology. The purpose of image region segmentation is to divide the region of an object from the image, that is, to find out those pixel sets corresponding to the object or the surface of the object. The pixel sets appear as two-dimensional clumps, which is one of the basic shape characteristics of the region.

The image that appears in the present disclosure includes, but is not limited to, the image data obtained after one or more of operations such as cropping, contrast adjusting, median filtering, and adaptive threshold segmentation performed on the original image, and may be either a JPEG image in the traditional sense or a image in other formats, or may be other types of pixels that have been subjected to image processing, as long as the technical purpose aimed to be realized by the technical solutions proposed in this disclosure can be achieved.

The term "first intermediate image" in the present disclosure refers to a non-ultraviolet light intermediate image that only includes teeth and at least one portion of a gum region of a non-ultraviolet light image.

The term "color space" in the present disclosure basically refers to a color model (also referred to as a color space or a color system), and its purpose is to describe colors in a generally acceptable manner under a certain standard. Where, the term "RGB color space" basically refers to the color space based on the three basic colors of R (Red), G (Green), and B (Blue), which are superimposed in different degrees to produce numerous colors in a wide range, and thus it is commonly known as the three primary color mode. The term "HSV color space" basically refers to a color space proposed for better digital processing of colors. There are many HSX color spaces, where X may be V or I, and X has different meanings depending on the specific use. H is hue, S is saturation, V is lightness, and I is intensity. The term "LAB color space" basically refers that it consists of three elements, where one element is brightness (L), and a and b are two color channels. a includes colors ranging from dark green (low brightness value) to gray (medium brightness value) and then to bright pink (high brightness value); and b is from bright blue (low brightness value) to gray (medium brightness value) and then to yellow (high brightness value). Therefore, this color mixing will produce bright colors.

The term "second intermediate image" in the present disclosure refers to an ultraviolet light intermediate image including only the tooth region.

The term "plaque comparison threshold" in the present disclosure basically refers to a threshold interval including a high threshold and a low threshold, where the low threshold is lower than the high threshold, and the low threshold and the high threshold are selected from the following range: np.array([3, 45, 50]) to np.array([25, 250, 88]).

Before starting to introduce the oral cavity detection method proposed in the present disclosure, the applicant of the present disclosure hopes to first introduce the design of hardware selection of the oral cavity detection device disclosed in the present disclosure, but the following description is only exemplary rather than limiting. Other selections are also feasible as long as the intended purpose of the design can be achieved. Namely, other selections will also fall within the scope as claimed to be protected by the claims of the present disclosure.

In terms of the hardware, through the following specially designed structure, high-quality non-UV such as white light photos and UV photos of the oral cavity can be taken. Where the camera device is for example an 8 million HD autofocus module, of Model KS8A17, with a module size of 38 mm x 38 mm, an operating temperature of -20°C to 70°C, and an imaging distance of 3 cm to infinity. This module can provide a 3264 x 2448 resolution photo, has excellent responsiveness in a low-light environment, and can provide high-speed transmission to a controller for processing. The ultraviolet light module may be, for example, a UV lamp structure. The UV lamp has a simple appearance, including UV lamp electrodes, built-in UV lamp beads (with a central wavelength of for example 365 nm), a cone-shaped structure (which can achieve a light-gathering function), and a UV filter (which can filter out the interference light and remain only the UV light to pass through). After powered, the module can serve as a UV light source in the acquisition process of an oral cavity image. As for the arrangement of the UV lamps, the UV lamp part can be composed of, for example, two UV lamps, which are respectively located on the left and right sides of the camera, with a distance of 1 to 3 cm from the camera.

In addition, the non-ultraviolet light module may be, for example, a white light LED lamp structure, with 3 mm LED white light emitting diodes. As for the arrangement of LED lamps, the LED lamp part is composed of, for example, four white LED lamps, which are respectively located at the 4 top corners of the oral cavity detector, and are arranged in a rectangular shape around the camera, with a distance between 1 and 5 cm from the camera.

Furthermore, the power supply module used can supply power to the UV lamp. Two UV lamps can be powered respectively by two AA batteries with a voltage of 1.5 V. The battery and the UV lamp are connected through a relay, to achieve control. In addition, the power supply module may also include a module for powering the controller. A 5 V 1500 mAh lithium battery can serve as the power supply for the whole machine, and structures except the UV lamp is powered by it.

In terms of algorithms, the traditional method of analyzing dental plaque by ultraviolet light only adopts ultraviolet ultraviolet light images for analysis, and the oral cavity images under ultraviolet light are presented in a fluorescent color. At this moment, teeth and gums both exhibit a fluorescent color and cannot be distinguished well, leading to inaccurate detection. Based on the high-quality non-UV photos such as white light photos and UV photos of the oral cavity acquired based on the hardware, the inventors of the present disclosure conducted the following analysis on oral issues. Namely, the inventors of the present disclosure creatively thought of using the region including teeth and at least one portion of a gum determined by the ultraviolet light image to analyze the non-ultraviolet light image, then obtaining the region only comprising the teeth, and then analyzing the plaque condition of the subject by the determined tooth region in combination with the ultraviolet light image. Here, preferably, the objects captured by the ultraviolet light image and the non-ultraviolet light image are the same.

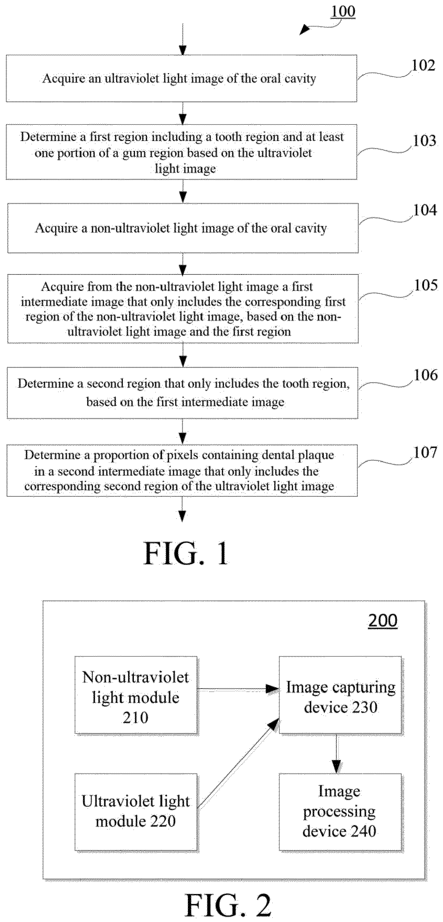

FIG. 1 shows a flowchart of an oral cavity detection method 100 according to an embodiment of the present disclosure. As can be seen from FIG. 1 that, the oral cavity detection method 100 according to an embodiment of the present disclosure comprises the following steps. Firstly, in Step 102 of the method, an ultraviolet light image of the oral cavity is acquired; then, in Step 103 of the method, a first region including a tooth region and at least one portion of a gum region is determined based on the ultraviolet light image; then, in Step 104 of the method, a non-ultraviolet light image of the oral cavity is acquired; next, in Step 105 of the method, a first intermediate image that only includes the corresponding first region of the non-ultraviolet light image is acquired from the non-ultraviolet light image, based on the non-ultraviolet light image and the first region; then, in Step 106 of the method, a second region that only includes the tooth region is determined, based on the first intermediate image; and finally, in Step 107 of the method, the proportion of pixels containing dental plaque in a second intermediate image that only includes the corresponding second region of the ultraviolet light image is determined.

For example, during the detection of dental plaque, a median filtering operation is performed on the ultraviolet light image of the oral cavity and the ultraviolet light image is converted into a gray-scale image; then an adaptive threshold segmentation operation is performed to determine the first region, which is an region including the teeth and at least one portion of a gum and is referred to herein as mask region mask1, then a non-ultraviolet light image including only the teeth and the at least one portion of the gum region is obtained through the use of the intersection of said mask1 and the non-ultraviolet light image, then the obtained ultraviolet light image is analyzed to obtain, for example, mask region mask2 that only includes the tooth region, and then, by taking the intersection of said mask2 and the ultraviolet light image, an ultraviolet light image including only the tooth region is obtained so that adverse effects of the gum region are eliminated during the detection of dental plaque.

More specifically, the determination of the first region including the tooth region and the at least one portion of the gum region based on the ultraviolet light image further comprises: performing a median filtering operation on the ultraviolet light image and converting the ultraviolet light image into a gray-scale image; and performing an adaptive threshold segmentation operation to determine the first region. That is to say, after the ultraviolet light image is obtained, a median filtering operation will be performed on the ultraviolet light image, thereby to eliminate noise points. For example, if a 3*3 pixel matrix is taken in the image, that is, there are 9 pixel points in the pixel matrix, the 9 pixels are ordered, and finally the center point of this matrix is assigned to the median value of these 9 pixels. This operation is refered to as a median filtering operation. Then it is converted to a gray-scale image. In the specific conversion method, each pixel point in a three-channel image of RGB three colors is subjected to the operation of r*0.2126+g*0.7152+b*0.0722, and three-channel images are proportionally superimposed, to obtain the gray-scale image. Next, adaptive threshold adjustment and region segmentation are performed, to divide out the region of interest. Specifically, the actual value of each pixel is distributed between [0, 255] on the basis of the gray-scale image, and then it is needed to further subdivide those pixel points as the foreground and the background. The adaptation here is to traverse from 0 to 255, to determine whether a pixel value is a foreground pixel point or a background pixel point such that the correlation coefficient of the class of foreground pixel points and the class of background pixel points are maximized, thereby to obtain an adaptive threshold, and then the adaptive threshold is used to distinguish the foreground pixels from the background pixels and perform the region segmentation operation so as to obtain the region of interest, which is here only the tooth region and the at least one portion of the gum region. Optionally, the determination of the first region including the tooth region and the at least one portion of the gum region based on the ultraviolet light image may further comprise adjusting the contrast of the ultraviolet light image, to enhance the contrast. In short, for example, through the use of an original image and a black image, pixels are superimposed proportionally to form a new image. The difference between the pixel values of the foreground and the background of the new image is increased, i.e., the image contrast is enhanced, thereby facillitating a subsequent adaptive threshold segmentation operation.

Where, Step 107 of the method is determining the proportion of pixels containing dental plaque in a second intermediate image that only includes the corresponding second region of the ultraviolet light image, further comprising: converting the second intermediate image from an RGB color space to an HSV color space; receiving a dental plaque comparison threshold; and determining the proportion of pixels containing dental plaque based on the second intermediate image converted to the HSV color space and the dental plaque comparison threshold.

For example, the second intermediate image is first converted from an RGB color space to an HSV color space, in which the identification of dental plaque can be facilitiated. Next, a dental plaque comparison threshold is received. Here, the dental plaque comparison threshold may be preset or obtained based on big data analysis. Here, for example, the low threshold is np.array([3, 45, 50]) and the high threshold is np.array([25, 250, 88]). Here, those skilled in the art should understand that, the selection of the low threshold and the high threshold here is only exemplary rather than restrictive, and other threshold selections that can solve the problems that the present disclosure aims to solve and achieve the corresponding technical effects shall also fall within the scope of as claimed to be protected by the claims of the present disclosure. Then the pixel region of suspected dental plaque is obtained from this interval, and the proportion of pixels containing dental plaque is determined based on the second intermediate image converted to the HSV color space and the dental plaque comparison threshold. Furthermore, a ratio of these detected pixel points to the total number of pixel points in the detection region can be used to obtain a plaque detection score.

Correspondingly, the determination of the second region that only includes the tooth region based on the first intermediate image further comprises: performing a median filtering operation on the first intermediate image and converting the first intermediate image into a gray-scale image; and performing an adaptive threshold segmentation operation to determine the second region. Where, the non-ultraviolet light may be white light or yellow light. That is to say, after the first intermediate image is obtained, a median filtering operation will be performed on the first intermediate image to thereby eliminate noise points. For example, if a 3*3 pixel matrix is taken in the image, that is, there are 9 pixel points in the pixel matrix, the 9 pixels are ordered, and finally the center point of this matrix is assigned to the median value of these 9 pixels. This operation is referred to as a median filtering operation. Then it is converted to a gray-scale image. In the specific conversion method, each pixel point in a three-channel image of RGB three colors is subjected to the operation of r*0.2126+g*0.7152+b*0.0722, and three-channel images are proportionally superimposed, to obtain the gray-scale image. Next, adaptive threshold adjustment and region segmentation are performed, to divide out the region of interest. Specifically, the actual value of each pixel is distributed between [0, 255] on the basis of the gray-scale image, and then it is needed to further subdivide those pixel points as the foreground and the background. The adaptation here is to traverse from 0 to 255, to determine whether a pixel value is a foreground pixel point or a background pixel point such that the correlation coefficient of the class of foreground pixel points and the class of background pixel points are maximized, thereby to obtain an adaptive threshold, and then the adaptive threshold is used to distinguish the foreground pixels from the background pixels and perform the region segmentation operation so as to obtain the region of interest, which is here only the tooth region. Preferably, the determination of the second region that only includes the tooth region based on the first intermediate image further comprises: adjusting the contrast of the first intermediate image, to enhance the contrast. In short, for example, through the use of an original image and a black image, pixels are superimposed proportionally to form a new image. The difference between the pixel values of the foreground and the background of the new image is increased, i.e., the image contrast is enhanced, thereby facilitating a subsequent adaptive threshold segmentation operation.

Through the combined use of ultraviolet light and non-ultraviolet light images, the oral cavity detection method proposed by one embodiment according to the present disclosure can accurately determine teeth and junction regions between teeth and gums, and thereby can more determine the proportion of pixels containing dental plaque, so as to provide a favorable basis for dental plaque assessment.

FIG. 2 shows another embodiment of an oral cavity detection device 200 disclosed according to the present disclosure. As can be seen from FIG. 2 that, the oral cavity detection device comprises: a non-ultraviolet light module 210, configured to emit non-ultraviolet light; an ultraviolet light module 220, configured to emit ultraviolet light; a camera device 230, configured to acquire a non-ultraviolet light image of the oral cavity and an ultraviolet light image of the oral cavity; and a image processing device 240, configured to determine the proportion of pixels containing dental plaque based on the non-ultraviolet light image and the ultraviolet light image.

In one embodiment according to the present disclosure, the image processing device 240 is further configured to:
acquire an ultraviolet light image of the oral cavity;
determine a first region including a tooth region and at least one portion of a gum region based on the ultraviolet light image;
acquire a non-ultraviolet light image of the oral cavity;
acquire from the non-ultraviolet light image a first intermediate image that only includes the corresponding first region of the non-ultraviolet light image, based on the non-ultraviolet light image and the first region;
determine a second region that only includes the tooth region, based on the first intermediate image; and
determine the proportion of pixels containing dental plaque in a second intermediate image that only includes the corresponding second region of the ultraviolet light image.

In one embodiment according to the present disclosure, the determination of the proportion of pixels containing dental plaque in the second intermediate image that only includes the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

In one embodiment according to the present disclosure, the determination of the first region including the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

In one embodiment according to the present disclosure, the determination of the first region including the tooth region and the at least one portion of the gum region based on the ultraviolet light image further comprises:
adjusting the contrast of the ultraviolet light image.

In one embodiment according to the present disclosure, the determination of the second region that only includes the tooth region based on the first intermediate image further comprises:
performing a median filtering operation on the first intermediate image and converting the first intermediate image into a gray-scale image; and
performing an adaptive threshold segmentation operation to determine the second region.

In one embodiment according to the present disclosure, the determination of the second region that only includes the tooth region based on the first intermediate image further comprises:
adjusting the contrast of the first intermediate image.

In one embodiment according to the present disclosure, the non-ultraviolet light is white light or yellow light.

An embodiment of the present disclosure further proposes a tangible computer-readable storage medium, where the storage medium includes an instruction for executing the oral cavity detection method, and when the instruction is executed, a processor of the computer is configured at least to:
acquire an ultraviolet light image of the oral cavity;
determine a first region including a tooth region and at least one portion of a gum region based on the ultraviolet light image;
acquire a non-ultraviolet light image of the oral cavity;
acquire from the non-ultraviolet light image a first intermediate image that only includes the corresponding first region of the non-ultraviolet light image, based on the non-ultraviolet light image and the first region;
determine a second region that only includes the tooth region, based on the first intermediate image; and
determine the proportion of pixels containing dental plaque in a second intermediate image that only includes the corresponding second region of the ultraviolet light image.

In one embodiment according to the present disclosure, the determination of the proportion of pixels containing dental plaque in the second intermediate image that only includes the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

In one embodiment according to the present disclosure, the determination of the first region including the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

In one embodiment according to the present disclosure, the determination of the first region including the tooth region and the at least one portion of the gum region based on the ultraviolet light image further comprises:
adjusting the contrast of the ultraviolet light image.

In one embodiment according to the present disclosure, the determination of the second region that only includes the tooth region based on the first intermediate image further comprises:
performing a median filtering operation on the first intermediate image and converting the first intermediate image into a gray-scale image; and
performing an adaptive threshold segmentation operation to determine the second region.

In one embodiment according to the present disclosure, the determination of the second region that only includes the tooth region based on the first intermediate image further comprises:
adjusting the contrast of the first intermediate image.

In one embodiment according to the present disclosure, the non-ultraviolet light is white light or yellow light.

In addition, alternatively, the method described above may be implemented by a computer program product, i.e., a computer-readable storage medium. The computer program product may include a computer-readable storage medium, carrying computer-readable program instructions thereon for performing various aspects of the present disclosure. The computer-readable storage medium may be tangible equipment that can hold and store instructions for use by instruction execution equipment. The computer-readable storage medium may be, for example, but is not limited to, electrical storage equipment, magnetic storage equipment, optical storage equipment, electromagnetic storage equipment, semiconductor storage equipment, or any suitable combination of the foregoing. More specific examples (the list of which is not exhaustive) of the computer-readable storage medium include: a portable computer diskette, a hard disk, a random access memory (RAM), a read-only memory (ROM), an erasable programmable read-only memory (EPROM or flash memory), a static random access memory (SRAM), a portable compact disc read-only memory (CD-ROM), a digital versatile disc (DVD), a memory stick, a floppy disk, mechanical coding equipment, a punched card or protruding structure in a groove, for example, having instructions stored thereon, and any suitable combination of the foregoing. The computer-readable storage medium used herein is not to be construed as an instantaneous signal per se, such as a radio wave or another freely propagating electromagnetic wave, an electromagnetic wave propagating through a waveguide or other transmission medium (e.g., a light pulse passing through a fiber optic cable), or an electrical signal transmitted through a wire.

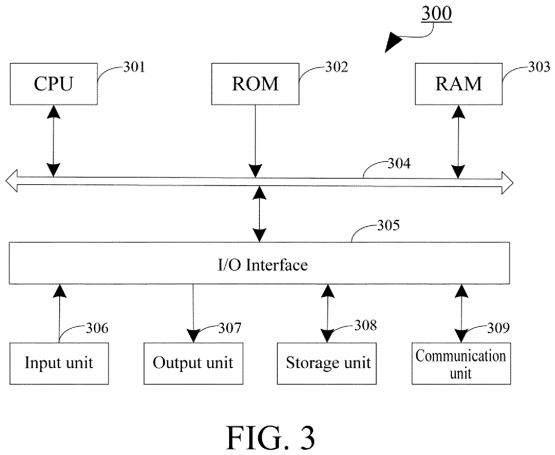

FIG. 3 shows a schematic block diagram of an oral cavity detection device 300 proposed by one embodiment according to the present disclosure. It should be understood that, the oral cavity detection device 300 may be implemented to achieve functions of the oral cavity detection method 100 in FIG. 1. It can be seen from FIG. 4 that, the oral cavity detection device 300 includes a central processing unit (CPU) 301 (e.g., a processor), which can perform various appropriate actions and processes according to computer program instructions stored in the read only memory (ROM) 302 or computer program instructions loaded from the storage unit 4308 into the random access memory (RAM) 303. In RAM 303, various programs and data required for the operation of the oral cavity detection device 300 may also be stored. CPU 301, ROM 302, and RAM 303 are connected to each other through bus 304. Input/output (I/O) interface 305 is also connected to the bus 304.

Multiple components in the oral cavity detection device 300 are connected to the I/O interface 305, and they include: input unit 306, such as a keyboard, and a mouse; output unit 307, such as various types of displays and speakers; and storage unit 308, such as a disk and an optical disc; and communication unit 309, such as a network card, a modem, and a wireless communication transceiver. The communication unit 309 allows the device 300 to exchange information/data with other equipment through a computer network such as the Internet and/or various telecommunication networks.

Generally speaking, a third aspect of the present disclosure provides a tangible computer-readable storage medium, where the storage medium includes an instruction for executing the oral cavity detection method, and when the instruction is executed, a processor of the computer is configured at least to perform functions of the oral detection method 100 according to FIG. 1.

The various methods described above, for example, the oral cavity detection method 100 may be executed by processing unit 301. For example, in some embodiments, the oral cavity detection method 100 may be implemented as a computer software program, which is tangibly comprised in a machine-readable medium, such as storage unit 308. In some embodiments, part or all of the computer program may be loaded and/or installed on the oral cavity detection device 300 via ROM 302 and/or communication unit 309. When the computer program is loaded into RAM 303 and executed by the processor/CPU 301, one or more actions or steps in the oral cavity detection method 100 described above can be executed.

Generally speaking, various exemplary embodiments of the present disclosure may be implemented in hardware or dedicated circuitry, software, firmware, logic, or any combination thereof. Some aspects may be implemented in hardware, while other aspects may be implemented in firmware or software that may be executed by a controller, a microprocessor, or other computing equipment. While aspects of embodiments of the present disclosure are illustrated or described as block diagrams, flowcharts, or represented using some other graphical representations, it will be understood that the blocks, devices, systems, techniques, or methods described herein may be implemented as non-limiting examples in hardware, software, firmware, dedicated circuits or logic, general-purpose hardware or controllers, or other computing equipment, or some combination thereof.

Although it has been described above that various exemplary embodiments of the present disclosure can be implemented in hardware or dedicated circuits, the aforementioned data processing equipment for the block chain can be implemented in the form of hardware or software. This is because: in the 1990s, a technical improvement can easily be a hardware improvement (for example, an improvement in circuit structures such as diodes, transistors, and switches) or a software improvement (for example, an improvement in method flows). However, with continued development of technology, many improvements in method flows today can almost be achieved by programming an improved method flow into a hardware circuit. In other words, a corresponding hardware circuit structure is obtained by programming different programs for the hardware circuit, that is, the hardware circuit structure is changed. Therefore, such improvements in method flows can also be regarded as direct improvements in the hardware circuit structure. Therefore, it cannot be said that an improvement in a method flow cannot be implemented by a hardware entity module. For example, a programmable logic device (PLD) (such as a field programmable gate array (FPGA)) is an integrated circuit whose logic function is determined by programming the device by a user. The designer performs programming by himself to "integrate" a digital system on a piece of programmable logic device, without the need for a dedicated integrated circuit chip designed and manufactured by a chip manufacturer. Also, instead of making an integrated circuit chip manually, this programming is also implemented with "logic compiler" software similar to the software compiler used in program development writing, while the raw code to be compiled has to be written in a particular programming language, referred to as the Hardware Description Language (HDL), and HDL also does not have only one kind, but rather multiple kinds, such as Advanced Boolean Expression Language (ABEL), Altera Hardware Description Language (AHDL), Confluence, Cornell University Programming Language (CUPL), HDCal, Java Hardware Description Language (JHDL), Lava, Lola, MyHDL, PALASM, Ruby Hardware Description Language (RHDL), etc., The Very-High-Speed Integrated Circuit Hardware Description Language (VHDL) and Verilog are currently most commonly used. It should also be clear to those skilled in the art that the hardware circuit for implementing the logic method flow can be easily obtained by only slightly logically programming a method flow using the above several hardware description languages and programming the method flow into an integrated circuit.

Computer-readable program instructions or computer program products for executing various aspects of the present disclosure can also be stored in the cloud. When invoking is required, a user can access the computer-readable program instructions stored on the cloud for executing an aspect of the present disclosure via a mobile Internet, a solid network, or other networks, thereby implementing the technical solutions disclosed according to various aspects of the present disclosure.

In summary, through the combined use of ultraviolet light and non-ultraviolet light images, the oral cavity detection method, the oral cavity detection device and correspondingly the computer-readable storage medium provided by the three aspects of the present disclosure can accurately determine teeth andjunction regions between teeth and gums, and thereby can accurately determine the proportion of pixels containing dental plaque, so as to provide a favorable basis for dental plaque assessment.

The above descriptions are merely optional embodiments of the present disclosure, and are not intended to limit the embodiments of the present disclosure. For those skilled in the art, the embodiments of the present disclosure may have various changes and modifications. Any modifications, equivalent substitutions, improvements, etc. made within the spirit and principle of the embodiments of the present disclosure shall fall within the protection scope of the embodiments of the present disclosure.

While embodiments of the present disclosure have been described with reference to several specific embodiments, it should be understood that embodiments of the disclosure are not limited to the specific embodiments disclosed. Embodiments of the present disclosure are intended to cover various modifications and equivalent arrangements included within the spirit and scope of the appended claims. The scope of the claims is accorded the broadest interpretation, thereby including all such modifications as well as equivalent structures and functions.

## Claims

1. An oral cavity detection method, wherein the oral cavity detection method comprises:
acquiring an ultraviolet light image of the oral cavity;
determining a first region comprising a tooth region and at least one portion of a gum region based on the ultraviolet light image;
acquiring a non-ultraviolet light image of the oral cavity;
acquiring from the non-ultraviolet light image a first intermediate image that only comprises the corresponding first region of the non-ultraviolet light image, based on the non-ultraviolet light image and the first region;
determining a second region that only comprises the tooth region, based on the first intermediate image; and
determining the proportion of pixels containing dental plaque in a second intermediate image that only comprises the corresponding second region of the ultraviolet light image.

2. The oral cavity detection method according to claim 1, wherein the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

3. The oral cavity detection method according to claim 1, wherein the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

4. The oral cavity detection method according to claim 3, wherein the determination of the first region comprising the tooth region and the at least one portion of the gum region based on the ultraviolet light image further comprises:
adjusting the contrast of the ultraviolet light image.

5. The oral cavity detection method according to claim 1, wherein the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

6. The oral cavity detection method according to claim 5, wherein the determination of the second region that only comprises the tooth region based on the first intermediate image further comprises:
adjusting the contrast of the first intermediate image.

7. The oral cavity detection method according to claim 1, wherein the non-ultraviolet light is white light or yellow light.

8. An oral cavity detection device, **characterized by** comprising:
a non-ultraviolet light module, configured to emit non-ultraviolet light;
an ultraviolet light module, configured to emit ultraviolet light;
an image capturing device, configured to acquire a non-ultraviolet light image of the oral cavity and an ultraviolet light image of the oral cavity; and
a image processing device, configured to determine the proportion of pixels containing dental plaque based on the non-ultraviolet light image and the ultraviolet light image.

9. The oral cavity detection device according to claim 8, wherein the image processing device is further configured to:
acquire an ultraviolet light image of the oral cavity;
determine a first region comprising a tooth region and at least one portion of a gum region based on the ultraviolet light image;
acquire a non-ultraviolet light image of the oral cavity;
acquire from the non-ultraviolet light image a first intermediate image that only comprises the corresponding first region of the non-ultraviolet light image, based on the non-ultraviolet light image and the first region;
determine a second region that only comprises the tooth region, based on the first intermediate image; and
determine the proportion of pixels containing dental plaque in a second intermediate image that only comprises the corresponding second region of the ultraviolet light image.

10. The oral cavity detection device according to claim 9, wherein the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

11. The oral cavity detection device according to claim 9, wherein the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

12. The oral cavity detection device according to claim 11, wherein the determination of the first region comprising the tooth region and the at least one portion of the gum region based on the ultraviolet light image further comprises:
adjusting the contrast of the ultraviolet light image.

13. The oral cavity detection device according to claim 9, wherein the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

14. The oral cavity detection device according to claim 13, wherein the determination of the second region that only comprises the tooth region based on the first intermediate image further comprises:
adjusting the contrast of the first intermediate image.

15. The oral cavity detection device according to claim 8, wherein the non-ultraviolet light is white light or yellow light.

16. A tangible computer-readable storage medium, wherein the storage medium comprises an instruction for executing the oral cavity detection method, and when the instruction is executed, a processor of the computer is configured at least to:
acquire an ultraviolet light image of the oral cavity;
determine a first region comprising a tooth region and at least one portion of a gum region based on the ultraviolet light image;
acquire a non-ultraviolet light image of the oral cavity;
acquire from the non-ultraviolet light image a first intermediate image that only comprises the corresponding first region of the non-ultraviolet light image, based on the non-ultraviolet light image and the first region;
determine a second region that only comprises the tooth region, based on the first intermediate image; and
determine the proportion of pixels containing dental plaque in a second intermediate image that only comprises the corresponding second region of the ultraviolet light image.

17. The computer-readable storage medium according to claim 16, wherein the determination of the proportion of pixels containing dental plaque in the second intermediate image that only comprises the corresponding second region of the ultraviolet light image further comprises:
converting the second intermediate image from an RGB color space to an HSV color space;
receiving a dental plaque comparison threshold; and
determining the proportion of pixels containing dental plaque based on the second intermediate image converted to the HSV color space and the dental plaque comparison threshold.

18. The computer-readable storage medium according to claim 16, wherein the determination of the first region comprising the tooth region and the at least one portion of the gum region based on the ultraviolet light image further comprises:
performing a median filtering operation on the ultraviolet light image and converting the ultraviolet light image into a gray-scale image; and
performing an adaptive threshold segmentation operation to determine the first region.

19. The computer-readable storage medium according to claim 18, wherein the determination of the first region comprising the tooth region and the at least one portion of the gum region based on the ultraviolet light image further comprises:
adjusting the contrast of the ultraviolet light image.

20. The computer-readable storage medium according to claim 16, wherein the determination of the second region that only comprises the tooth region based on the first intermediate image further comprises:
performing a median filtering operation on the first intermediate image and converting the first intermediate image into a gray-scale image; and
performing an adaptive threshold segmentation operation to determine the second region.

21. The computer-readable storage medium according to claim 20, wherein the determination of the second region that only comprises the tooth region based on the first intermediate image further comprises:
adjusting the contrast of the first intermediate image.

22. The computer-readable storage medium according to claim 16, wherein the non-ultraviolet light is white light or yellow light.
